# EUROPÄISCHE PATENTANMELDUNG

(11) **EP 2 944 281 A1**
(43) Veröffentlichungstag der Anmeldung: **18.11.2015**
(21) Anmeldenummer: 15164584.3
(22) Anmeldetag: 22.04.2015
(51) Int. Cl.: A61B 18/14, A61N 1/05

(54) **ELEKTRODENELEMENT ZUR ELEKTROMEDIZINISCHEN THERAPIE IN EINEM MENSCHLICHEN ODER TIERISCHEN KÖRPER**

(30) Priorität: 13.05.2014 US 201461992248 P
(71) Anmelder: Biotronik AG, 8180 Bülach (CH)
(72) Erfinder: Richter, Jan Helge, 12355 Berlin (DE); Kolberg, Gernot, 12161 Berlin (DE)
(74) Vertreter: Galander, Marcus

(57) **Zusammenfassung**

Die Erfindung betrifft ein Elektrodenelement (10) zur elektromedizinischen Therapie in einem menschlichen oder tierischen Körper, umfassend einen Elementkörper (12), an dem wenigstens ein elektrischer Kontakt (50) an einer äußeren Oberfläche (16) des Elementkörpers (12) angeordnet ist, wobei der wenigstens eine elektrische Kontakt (50) aus der Oberfläche (16) des Elementkörpers (12) hervorsteht. Der wenigstens eine elektrische Kontakt (50) weist einen erhabenen Kontaktkörper (52) mit einer Stirnseite (54) auf, auf der wenigstens eine metallische Fläche (56) ausgebildet ist, wobei die metallische Fläche (56) von einer elektrischen Isolierung (58) umgeben ist.

Ferner betrifft die Erfindung einen Katheter (100) sowie ein Stimulationsaggregat (200) mit einem Elektrodenelement (10).

## Beschreibung

Die Erfindung betrifft ein Elektrodenelement zur elektromedizinischen Therapie in einem menschlichen oder tierischen Körper sowie einen Katheter mit einem Elektrodenelement.

Aus der US 2011/0301676 Al ist ein Katheter mit einem Elektrodenelement zur elektromedizinischen Therapie bekannt, bei dem erhabene elektrische Kontakte an der Außenseite des Katheters angeordnet sind. Die elektrischen Kontakte sind ringförmig um den Katheter gelegt und können wandständig an das jeweilige zu behandelnde Gefäß gepresst werden.

Bei der elektromedizinischen Therapie wird ein Stromfluss zur Stimulation, Ablation und dergleichen durch ein in situ eingebrachtes Elektrodenelement eingesetzt.

Generell problematisch sind jedoch parasitäre Ströme, so genannte Shuntströme, die bei derartigen seitlich angeordneten elektrischen Kontakten besonders häufig auftreten und durch Stromfluss über das Blut im zu therapierenden Gebiet hervorgerufen werden. Shuntströme haben mehrere Nachteile. Sie machen zum einen das angewandte therapeutische System ineffizient, denn der Shuntstrom ist für die elektromedizinische Therapie verloren. Ein Schrittmacher z. B. muss mehr Energie aufbringen, als zur reinen elektromedizinischen Therapie erforderlich wäre. Zum anderen kann aus der Energiebilanz während der Therapie nicht direkt auf einen therapeutischen Erfolg geschlossen werden, da ein Teil der applizierten Energie nicht der Therapie zu Gute kommt. Ferner können Shuntströme zu einer Koagulation des Blutes führen.

Der Erfindung liegt die Aufgabe zugrunde, ein verbessertes Elektrodenelement sowie einen Katheter, eine Elektrode oder ein kabelloses implantierbares Gerät mit einem Elektrodenelement anzugeben, bei dem eine Shuntstromproblematik verringert ist, insbesondere für Herzelektroden, Nervenelektroden, implantierbare Sensoren, schnurlose Schrittmacher.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Es wird ein Elektrodenelement zur elektromedizinischen Therapie in einem menschlichen oder tierischen Körper vorgeschlagen, umfassend einen Elementkörper, an dem wenigstens ein elektrischer Kontakt an einer äußeren Oberfläche des Elementkörpers angeordnet ist, wobei der wenigstens eine elektrische Kontakt aus der Oberfläche des Elementkörpers hervorsteht. Der wenigstens eine elektrische Kontakt weist einen erhabenen Kontaktkörper mit einer Stirnseite auf, auf dem wenigstens eine metallische Fläche ausgebildet ist, wobei die metallische Fläche von einer elektrischen Isolierung umgeben ist.

Vorteilhaft ist durch die Struktur des Elektrodenelements die metallische Fläche leicht wandständig an eine körpereigene Wand, etwa eine Gefäßwand oder ein Organ, so anzulegen, dass die metallische Fläche durch die Wand abgedeckt ist. Die wenigstens eine metallische Fläche bildet die eigentliche Elektrode des Elektrodenelements. Durch die elektrische Isolierung, die um die wenigstens eine metallische Fläche herum angeordnet ist, ist ein Stromfluss außerhalb des Kontaktgebiets, den die metallische Fläche mit der Wand bildet, effektiv reduziert oder sogar unterbunden. Dabei kann die Isolierung in einer Ausführung durch eine elektrisch isolierende Beschichtung auf dem elektrischen Kontakt gebildet sein, oder in einer Ausführung durch eine elektrisch isolierende Abdeckung, die am elektrischen Kontakt befestigt ist, oder in einer Ausführung durch eine elektrisch isolierende Abdeckung, die nicht in unmittelbarem Körperkontakt mit dem elektrischen Kontakt steht, oder auch durch Kombinationen von wenigstens zweien der verschiedenen Ausführungen.

Neben der Energieeinsparung durch die Vermeidung von Shuntströmen kann leichter erkannt werden, ob das Elektrodenelement in gewünschter Weise am Zielobjekt, etwa einer Gefäßwand, einem Organ und dergleichen, anliegt, da bei Vorliegen von Wandständigkeit eine deutliche Impedanzänderung des Elektrodenelements zu registrieren ist. Die Impedanz bzw. die Impedanzänderung, kann also vom Anwender als eindeutiger Indikator für den Nachweis der Wandständigkeit interpretiert werden. Dies gilt insbesondere für die so genannte bipolare Ablation, bei der z.B. ein Herzgewebe behandelt wird, um dessen Reizleitungsbahnen zu unterbrechen. Vorteilhaft ist die parallele Ablation mit mehreren Elektrodenpaaren unter Verwendung nur eines Generators

Vorteilhaft kann eine elektrisch aktive Fläche auf den Teil des elektrischen Kontakts reduziert werden, der in gutem mechanischen Kontakt mit dem Gewebe steht. Des Weiteren kann die Flächenpressung der Elektrode an der Gewebewandung erhöht werden.

Günstige Werkstoffe für die Isolation und die metallische Fläche (Elektrode) sind biokompatible Materialien. Günstige Isolationswerkstoffe sind z. B. Aluminiumoxide, Siliciumcarbid, diamantähnlicher Kohlenstoff (DLC), Parylene, Silikone, Polyurethane, Polysulfone, Polycarbonate, Polypropylene, Polymethylmethacrylate, PTFE (Polytetrafluorethylen), ETFE (Ethylen-Tetrafluorethylen), Copolymere aus Silikonen und Polyurethanen oder auch Polyimid in geeigneten Schichtdicken. Die Isolationswandstärken der Randisolation können vorteilhaft zwischen 10 nm und 100 µm betragen.

Günstige Werkstoffe für die metallische Fläche sind z. B. Platin, Platin-Iridium-Legierungen. Wolfram, Tantal, Gold, Palladium, Stahl, MP35N, Elgiloy.

Gemäß einer günstigen Ausgestaltung können mindestens Kanten um die metallische Fläche mit der elektrischen Isolierung zumindest bereichsweise abgedeckt sein. Günstigerweise kann ebenso eine elektrisch aktive Fläche auf den Teil des elektrischen Kontakts reduziert werden, der in gutem mechanischen Kontakt mit dem Gewebe steht.

Gemäß einer günstigen Ausgestaltung kann die elektrische Isolierung der wenigstens einen metallischen Fläche des elektrischen Kontakts wenigstens bereichsweise durch den Elektrodenkörper gebildet sein. Optional kann eine Kantenisolierung auf dem elektrischen Kontakt auch mit einer Isolierung, die durch den Elektrodenkörper gebildet wird, kombiniert sein. Dies kann in einer Ausführung jeweils an einem einzelnen Kontakt der Fall sein. In einer anderen Ausführung können ein oder mehrere von elektrischen Kontakten auf dem Elektrodenkörper mit Kantenisolierung versehen sein und ein oder mehrere andere Kontakte über den Elektrodenkörper isoliert sein.

Gemäß einer günstigen Ausgestaltung kann der Kontaktkörper als Ring ausgebildet sein und um den Elementkörper gelegt sein. Insbesondere kann die elektrische Isolierung in Umfangsrichtung mindestens die Hälfte des Rings bedecken, so dass die metallische Fläche ein Ringsegment bildet.

Bei der Ausführung des elektrischen Kontaktkörpers als Ring, welcher mehr oder gleich 180° radial an seiner Oberfläche elektrisch isoliert ist, ist die verbleibende (elektrisch aktive) Fläche als Ringsegment ausgebildet. Dadurch werden parallele Verlustströme während der Therapie durch das Blut vermieden. Das Ringsegment, wie auch der ganze Ring, ist gegenüber dem Elementkörper bzw. Katheterschaft erhaben und kann so positioniert werden, dass die elektrisch aktive Fläche nach Aktivierung des Kontaktkörpers bzw. Katheterschaftes wandständig zum Gewebe liegt. Unter Aktivierung des Elementkörpers versteht man eine konstruktiv vorgesehene Vorzugsform des Elektrodenelements (z. B. Helixform), mit der der Kontaktkörper an die Gefäßwandung angedrückt wird. Da die Impedanz bei vollständiger Wandständigkeit ansteigt, weil jeder Strom durch das im Vergleich zum Blut hochohmigere Gewebe fließen muss, ist einerseits eine Überprüfung der Wandständigkeit durch Messung der Impedanz vor einer Energieapplikation möglich. Andererseits kann durch die Ermittlung der eingebrachten Energie die Effektivität während der Therapie beurteilt werden.

Gemäß einer günstigen Ausgestaltung kann der elektrische Kontakt an einer Stirnseite des Elementkörpers angeordnet sein. Insbesondere kann der Kontaktkörper aus der Stirnseite des Elementkörpers hervorstehen. Diese Ausgestaltung ist besonders für schnurlose Herzschrittmacher ("leadless pacer") geeignet.

Gemäß einer günstigen Ausgestaltung können wenigstens zwei elektrische Kontakte vorgesehen sein, die axial übereinander am Elementkörper angeordnet sind, wobei vorzugsweise zwei elektrische Kontakte unterschiedliche Polarität aufweisen können.

Gemäß einer günstigen Ausgestaltung können wenigstens zwei elektrische Kontakte vorgesehen sein, die axial und radial voneinander beabstandet sind, wobei vorzugsweise zwei elektrische Kontakte unterschiedliche Polarität aufweisen können. Eine gegenüber dem, beispielsweise schaftförmigen, Elementkörper bzw. einer Katheterwandung beliebig geformte und gleichzeitig erhöhte Elektrodenfläche, die an den Seiten elektrisch isoliert sein kann und nur an der zum Gewebe anliegenden Fläche elektrisch aktiv ist, ist gegenüber dem Blut weitgehend isoliert.

Gemäß einer günstigen Ausgestaltung kann ein Array von mehreren elektrischen Kontakten vorgesehen sein. Ein Array oder eine Reihe von vielen (mindestens zwei) erhabenen, ggf. zum Rand hin isolierten metallischen Flächen mit gleicher Polarität hat den vorteilhaften Effekt, dass sich die relativ kleinen Flächen gegenüber einer großen zusammenhängenden Fläche (Gefäßwand, Muskel, Organ etc.) sehr leicht in Wandständigkeit bringen lassen. Die Größe des Areals der metallischen Flächen bestimmt die Impedanz des Systems. Bei auseinanderliegenden einzelnen metallischen Flächen ist die Impedanz deutlich geringer als für die Summe der Impedanzen der einzelnen kleinen metallischen Flächen zu erwarten wäre. Für das Gewebe entsteht eine Elektrode (metallische Fläche), die großflächig wirkt, aber nur an wenigen Punkten anliegen muss. Die Shuntströme werden dadurch stark reduziert.

Gemäß einer günstigen Ausgestaltung kann der wenigstens eine elektrische Kontakt federnd gelagert sein. Die federnde Lagerung kann durch ein oder mehrere Federelemente, luftgepolstert oder flüssigkeitsgestützt bewirkt werden, so dass der wenigstens eine elektrische Kontakt auch bei Unebenheiten zuverlässig an das Gewebe angedrückt werden kann. Als Federelemente können beispielsweise Spiralfedern, Blattfedern oder flexibler Kunststoff vorgesehen sein.

Gemäß einer günstigen Ausgestaltung kann der wenigstens eine elektrische Kontakt mit einer Oberflächenstruktur ausgestattet sein, welche die effektive Kontaktfläche gegenüber makroskopischen geometrischen Abmessungen wenigstens verdoppelt. Die vergrößerte Oberfläche kann bewirkt werden, indem die metallische Fläche, z. B. fraktal beschichtet ist. Die vergrößerte Oberfläche bewirkt vorteilhaft, dass eine zwangsläufig vorhandene Doppelschichtkapazität des Elektrodenelements erheblich erhöht wird. Dadurch wird trotz hoher Ladungsverschiebung die Polarisation der Elektrodenfläche (metallische Fläche) sehr stark verringert. So können bei der elektromedizinischen Therapie große Ströme fließen, ohne dass es zu unerwünschten elektrolytischen Reaktionen kommt, die das Gewebe reizen würden. Außerdem können die Elektrodenflächen auch nach Strombeaufschlagung unmittelbar zur Ableitung geringer Muskelpotenziale genutzt werden, ohne dass diese von Polarisationseffekten überlagert werden.

Gemäß einer günstigen Ausgestaltung kann der Kontaktkörper wenigstens zwei getrennte metallische Flächen aufweisen, wobei vorzugsweise die zwei getrennten metallischen Flächen für unterschiedliche Polaritäten vorgesehen sind. Insbesondere kann die erste der Flächen im Wesentlichen eben ausgebildet sein und die zweite der metallischen Flächen über die erste metallische Fläche hinausragen. So kann vorteilhaft die stimulierende oder ablatierende Elektrode räumlich aus der Oberfläche des Elektrodenkörpers herausstehen, während die zweite (Gegen-)Elektrode in der Ebene der Wandung liegt.

Ein bipolarer elektrischer Kontakt, der sich als Scheibe auf dem Elementkörper bzw. dem Katheterschaft darstellt, in deren Mitte der eine Pol erhaben aus der Scheibe heraussteht, bewirkt, dass dieser Pol effektiv in das Gewebe gedrückt werden kann. Damit fließt jeglicher Strom zwangsläufig über das Gewebe. Der andere Pol kann sich als metallische Fläche in Höhe der Oberfläche des Elementkörpers bzw. der Katheteroberfläche befinden.

Gemäß einer günstigen Ausgestaltung kann der Kontaktkörper als Hülse ausgebildet sein, wobei die metallische Fläche einen Teil der Mantelfläche der Hülse bildet. Durch die "tonnenartige" Ausführung des Kontaktkörpers kann dessen elektrisch aktive Mantelfläche (metallische Fläche) radial aus dem Elementkörper bzw. Katheterschaft herausragen und so positioniert sein, dass die elektrisch aktive metallische Fläche nach Aktivierung des Elementkörpers bzw. Katheterschaftes wandständig zum Gewebe liegt. Dabei kann die Hülse im Bereich der herausragenden metallischen Fläche mit oder ohne Abflachung ausgebildet sein.

Gemäß einem weiteren Aspekt der Erfindung wird ein Katheter mit einem Elektrodenelement vorgeschlagen. Das Elektrodenelement kann mit dem Katheter verbunden sein oder in diesen integriert sein. Eine effiziente elektromedizinische Therapie ist möglich, da parasitäre Shuntströme durch das erfindungsgemäße Elektrodenelement wirksam verringert oder sogar unterbunden werden. Eine gegenüber der Katheterwandung beliebig geformte und gleichzeitig erhöhte Elektrodenfläche, die an den Seiten elektrisch isoliert sein kann und nur an der zum Gewebe anliegenden Fläche elektrisch aktiv ist, ist gegenüber dem Blut weitgehend isoliert.

Der elektrische Kontakt kann als "Knopf" ausgebildet sein, bei dem die elektrisch aktive Fläche ballig ausgebildet und gegenüber dem Elementkörper bzw. Katheterschaft erhaben ist. Der Knopf wird so positioniert, dass die elektrisch aktive metallische Fläche nach Aktivierung des Elementkörpers bzw. Katheterschaftes wandständig zum Gewebe liegt. Die Form des Kontaktkörpers und insbesondere der metallischen Fläche, welche die Elektrode bildet, kann je nach Bedarf ausgewählt werden, z. B. eckig, pilzförmig, als eckige Scheibe, als runde Scheibe.

Ferner wird ein Stimulationsaggregat mit einem erfindungsgemäßen Elektrodenelement vorgeschlagen. Das Stimulationsaggregat kann insbesondere als schnurloser Herzschrittmacher oder als Herzelektrode ausgebildet sein.

Nach einem vorteilhaften Verfahren zum Betreiben eines Elektrodenelements zur elektromedizinischen Therapie in einem menschlichen oder tierischen Körper mit elektrischer Energie, wobei das Elektrodenelement wenigstens einen elektrischen Kontakt aufweist, wird eine Impedanz des wenigstens einen elektrischen Kontakts zur Energieregelung bei der elektrischen Therapie verwendet. Vorteilhaft wird elektrische Energie nur dann in der geeigneten Höhe freigegeben, wenn wenigstens der eine elektrische Kontakt eine Mindestimpedanz aufweist. Die Impedanz kann neben oder anstatt den heute üblichen Parametern Temperatur oder/und Leistung zur Regelung verwendet werden.

Die Erfindung ist nachfolgend beispielhaft, anhand von in Zeichnungen dargestellten Ausführungsbeispielen, näher erläutert. Es zeigen in schematischer Darstellung:
- Fig. 1: eine isometrische Darstellung eines Teils eines Katheters mit einem Elektrodenelement mit ringförmigen elektrischen Kontakten als Elektroden;
- Fig. 2: ein einzelner ringförmiger elektrischer Kontakt aus Figur 1 mit metallischer Fläche als Kontaktfläche und elektrischer Isolation;
- Fig. 3: ein einzelner balliger elektrischer Kontakt mit stirnseitiger metallischer Fläche und randseitiger elektrischer Isolation;
- Fig. 4: eine isometrische Darstellung eines Teils eines Katheters mit einem Elektrodenelement mit den balligen elektrischen Kontakten als Elektroden aus Figur 3, die radial und axial an der Oberfläche des Elektrodenelements voneinander beabstandet sind;
- Fig.5: eine isometrische Darstellung eines Teils eines Katheters mit einem Elektrodenelement mit elektrischen Kontakten, die paarweise axial übereinander angeordnet sind, wobei die Paare radial voneinander beabstandet sind;
- Fig. 6: ein einzelner eckiger elektrischer Kontakt aus Figur 5 mit stirnseitiger metallischer Fläche und randseitiger elektrischer Isolation;
- Fig. 7: eine isometrische Darstellung eines Teils eines Katheters mit einem Elektrodenelement mit eckigen, aus einer Fläche vorstehenden elektrischen Kontakten, die axial übereinander angeordnet sind, wobei die Paare radial voneinander beabstandet sind;
- Fig. 8: ein einzelner eckiger elektrischer Kontakt aus Figur 7 mit stirnseitiger metallischer Fläche und randseitiger elektrischer Isolation;
- Fig. 9: eine isometrische Darstellung eines Teils eines Katheters mit einem Elektrodenelement mit runden, kugelförmigen, aus einer Fläche vorstehenden elektrischen Kontakten, die axial übereinander angeordnet sind, wobei die Paare radial voneinander beabstandet sind;
- Fig. 10: ein einzelner elektrischer Kontakt aus Figur 9 mit stirnseitiger metallischer Fläche und randseitiger elektrischer Isolation über einen metallischen Fläche;
- Fig. 11: eine isometrische Darstellung eines Teils eines Katheters mit einem Elektrodenelement mit runden, knopfförmigen elektrischen Kontakten, die paarweise axial übereinander angeordnet sind, wobei die Paare radial voneinander beabstandet sind;
- Fig. 12: ein einzelner elektrischer Kontakt aus Figur 11 mit stirnseitiger metallischer Fläche und randseitiger elektrischer Isolation;
- Fig. 13: eine isometrische Darstellung eines Teils eines Katheters mit einem Elektrodenelement mit hülsenförmigen elektrischen Kontakten, die axial übereinander angeordnet sind, wobei die Paare radial voneinander beabstandet sind;
- Fig. 14: ein einzelner elektrischer Kontakt aus Figur 13 mit umfangsseitiger metallischer Fläche und umfangsseitiger elektrischer Isolation;
- Fig. 15: ein alternativer einzelner elektrischer Kontakt, beispielsweise für eine Anordnung wie in Figur 13, mit abgeflachter umfangsseitiger metallischer Fläche und umfangsseitiger elektrischer Isolation; und
- Fig. 16: eine isometrische Darstellung eines Stimulationsaggregates mit einem bipolaren stirnseitig angebrachten Elektrodenelement mit zwei nebeneinander angeordneten metallischen Flächen.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

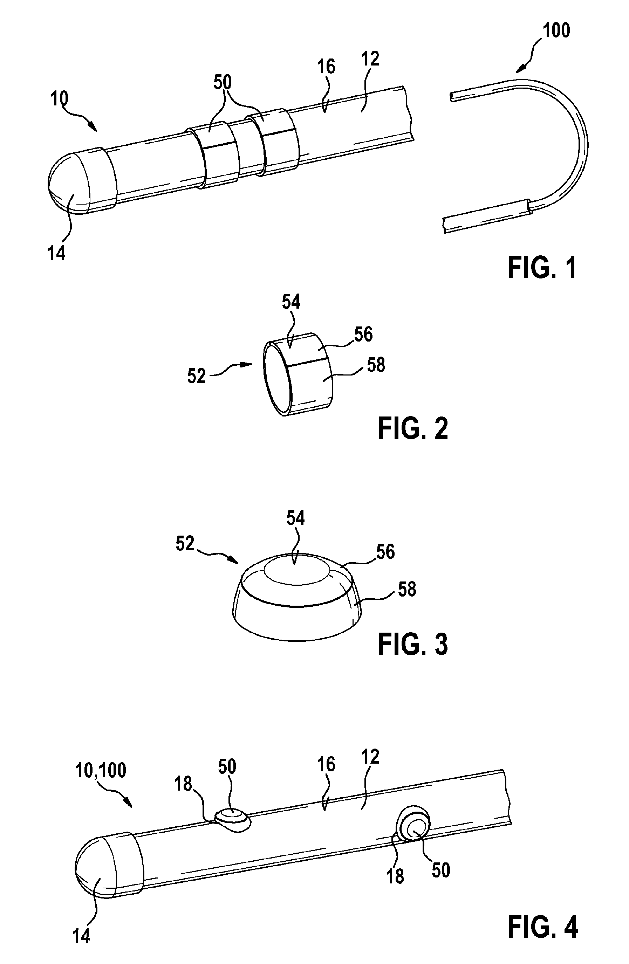

Figur 1 zeigt eine isometrische Darstellung eines Teils eines Katheters 100 mit einem Elektrodenelement 10 zur elektrischen Therapie in einem menschlichen oder tierischen Körper, mit ringförmigen elektrischen Kontakten 50 als Elektroden. Das Elektrodenelement 10 umfasst einen Elementkörper 12, der mit einer Spitze 14 abgeschlossen ist und an dem wenigstens ein elektrischer Kontakt 50 an einer äußeren Oberfläche 16 des Elementkörpers 12 angeordnet ist, wobei der wenigstens eine elektrische Kontakt 50 aus der Oberfläche 16 des Elementkörpers 12 hervorsteht. Der wenigstens eine elektrische Kontakt 50 weist einen erhabenen Kontaktkörper 52 mit einer Stirnseite 54 aufweist, auf dem eine metallische Fläche 56 ausgebildet ist, wobei die metallische Fläche 56 von einer elektrischen Isolierung 58 umgeben ist.

Figur 2 zeigt zur Illustration einen einzelnen ringförmigen elektrischen Kontakt 50 aus Figur 1 mit metallischer Fläche 56 als Kontaktfläche und elektrischer Isolation 58.

Figur 3 zeigt einen einzelnen elektrischen Kontakt 50 mit einem balligen Kontaktkörper 52, der an seiner Stirnseite 54 eine metallischer Fläche 56 aufweist, die randseitig mit einer elektrischen Isolation 58 umgeben ist.

Figur 4 zeigt eine isometrische Darstellung eines Teils eines Katheters 100 mit einem Elektrodenelement 10 zur elektrischen Therapie in einem menschlichen oder tierischen Körper, mit zwei balligen elektrischen Kontakten 50 aus Figur 3 als Elektroden. Das Elektrodenelement 10 umfasst einen Elementkörper 12, der mit einer Spitze 14 abgeschlossen ist und an dem die elektrischen Kontakte 50 an einer äußeren Oberfläche 16 des Elementkörpers 12 angeordnet sind. Die elektrischen Kontakte 50 stehen als Elektroden aus Öffnungen 18 aus der Oberfläche 16 des Elementkörpers 12 hervor. Die elektrischen Kontakte 50 sind voneinander radial und axial an der Oberfläche 16 des Elektrodenelements 10 beabstandet.

In den weiteren Ausführungsbeispielen sind die Elektrodenelemente 10 bis auf die elektrischen Kontakte 50 gleichartig aufgebaut, so dass nur auf die Unterschiede bei den Ausführungsbeispielen eingegangen wird. Alle gezeigten Kontaktkörper 52 erlauben eine sichere, wandständige Anlage der Elektrode an ein zu behandelndes Gebiet, z. B. eine Gefäßwand, wobei parasitäre Shuntströme vorteilhaft stark reduziert oder unterbunden werden können.

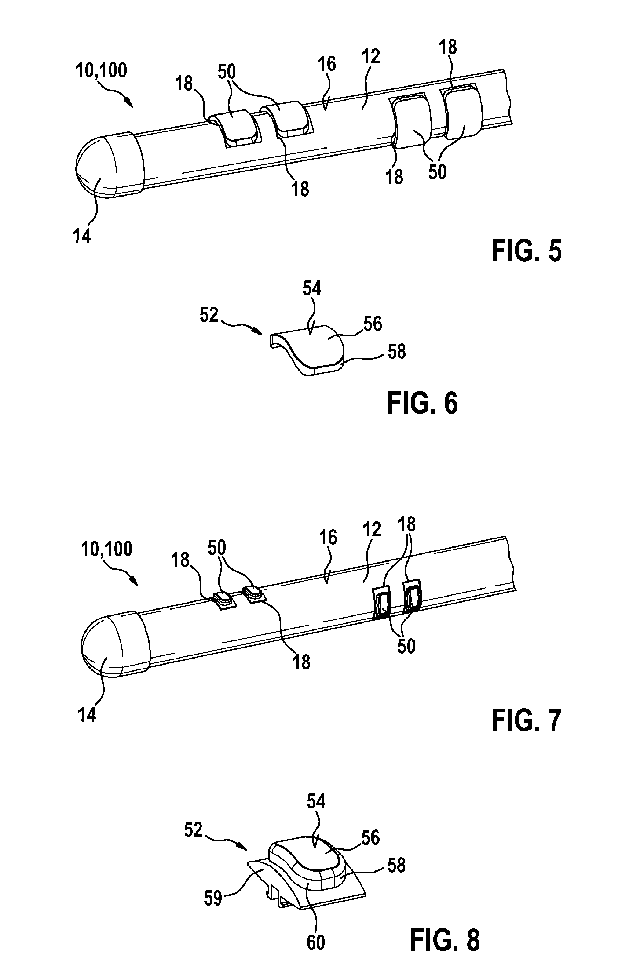

Figur 5 zeigt eine isometrische Darstellung eines Teils eines Katheters 100 mit einem Elektrodenelement 10 mit eckigen, aus einer Fläche 59 vorstehenden elektrischen Kontakten 50. Die elektrischen Kontakte 50 sind paarweise axial übereinander angeordnet, wobei die Paare radial voneinander beabstandet sind. Die elektrischen Kontakte 50 in jedem der Paare können jeweils unterschiedliche Polarität aufweisen.

Figur 6 zeigt einen einzelnen eckigen elektrischen Kontakt 50 aus Figur 5 mit metallischer Fläche 56 an der Stirnseite 54 und randseitiger elektrischer Isolation 58. Der Kontaktkörper 52 ist entsprechend einer Krümmung des Elementkörpers 12 gewölbt.

Figur 7 zeigt eine isometrische Darstellung eines Teils eines Katheters 100 mit einem Elektrodenelement 10 mit eckigen, aus einer Fläche 59 vorstehenden elektrischen Kontakten 50. Die elektrischen Kontakte 50 sind paarweise axial übereinander angeordnet, wobei die Paare radial voneinander beabstandet sind. Die elektrischen Kontakte 50 in jedem der Paare können jeweils unterschiedliche Polarität aufweisen.

Figur 8 zeigt einen einzelnen eckigen elektrischen Kontakt 50 aus Figur 7 mit metallischer Fläche 56 und randseitiger elektrischer Isolation 58. Der Kontaktkörper 52 weist eine Basisfläche 59 auf, aus der eine Erhebung 60 hervorsteht, an deren Stirnseite 54 die metallische Fläche 56 angeordnet ist.

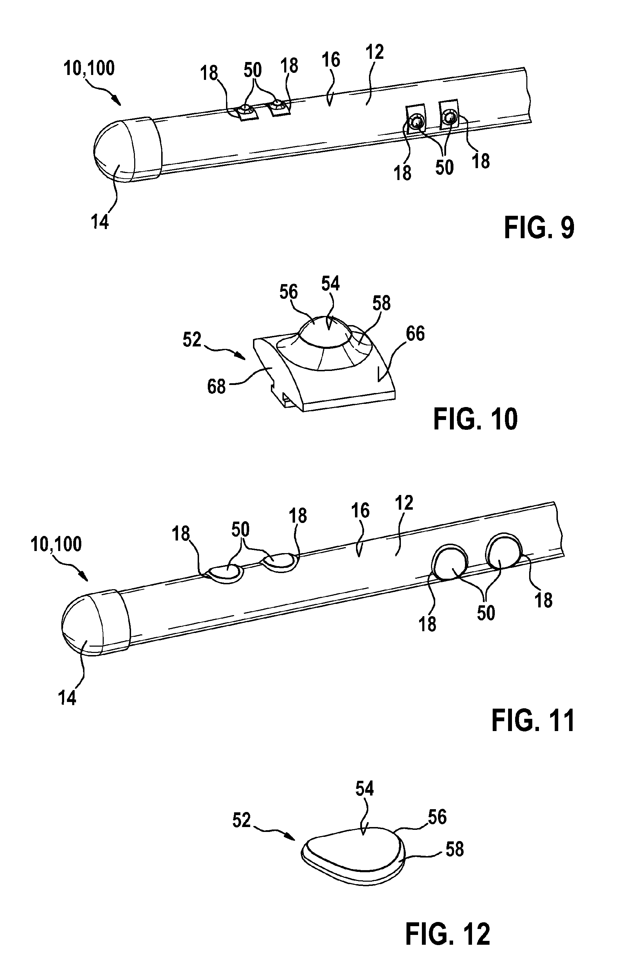

Figur 9 zeigt eine isometrische Darstellung eines Teils eines Katheters 100 mit einem Elektrodenelement 10 mit runden, kugelförmigen, aus einer Fläche vorstehenden elektrischen Kontakten 50. Die elektrischen Kontakte 50 sind paarweise axial übereinander angeordnet, wobei die Paare radial voneinander beabstandet sind. Jeder einzelne der elektrischen Kontakte 50 in jedem der Paare kann jeweils zwei Polaritäten aufweisen.

Figur 10 zeigt einen einzelnen elektrischen Kontakt 50 aus Figur 9 mit stirnseitiger, kugeliger metallischer Fläche 56 und randseitiger elektrischer Isolation 58. Der Kontaktkörper 52 weist eine ebene gewölbte, metallische Basisfläche 66 auf, über der sich die kugelige metallische Fläche 56 erhebt. Zwischen der Fläche 56 und der Basisfläche 66 ist die elektrische Isolation 58 ausgebildet. Die Basisfläche 66 ist ebenfalls von einer elektrischen Isolation 68 umgeben. Beide metallischen Flächen 56, 66 bilden unterschiedliche elektrische Pole, wobei die kugelige Fläche 56 im Einsatz z. B. direkt in das Myokard gedrückt werden kann und die Basisfläche 66 mit der Oberfläche 16 des Elektrodenelements 10 fluchten kann.

Figur 11 zeigt eine isometrische Darstellung eines Teils eines Katheters 100 mit einem Elektrodenelement 10 mit runden, flachen, gewölbten elektrischen Kontakten 50. Die elektrischen Kontakte 50 sind paarweise axial übereinander angeordnet, wobei die Paare radial voneinander beabstandet sind. Die elektrischen Kontakte 50 in jedem der Paare können jeweils unterschiedliche Polarität aufweisen.

Figur 12 zeigt einen einzelnen elektrischen Kontakt aus Figur 11 mit einer metallischen Fläche 56 an der Stirnseite 54 des Kontaktkörpers 52 und einer randseitigen elektrischen Isolation 58.

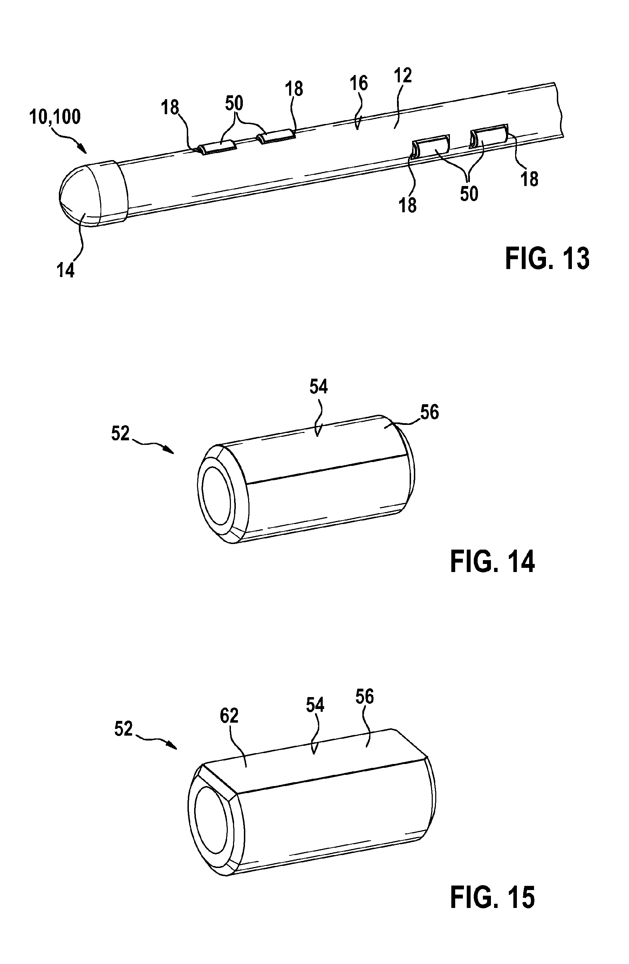

Figur 13 zeigt eine isometrische Darstellung eines Teils eines Katheters 100 mit einem Elektrodenelement 10 mit hülsenförmigen elektrischen Kontakten 50, bei denen ein jeweils ein Mantelsegment aus einer Öffnung 18 in der Oberfläche 16 des Elementkörpers 12 heraussteht. Die elektrischen Kontakte 50 sind paarweise axial übereinander angeordnet, wobei die Paare radial voneinander beabstandet sind. Die elektrischen Kontakte 50 in jedem der Paare können jeweils unterschiedliche Polarität aufweisen.

Figur 14 zeigt einen einzelnen hülsenförmigen elektrischen Kontakt 50 aus Figur 13 mit umfangsseitiger metallischer Fläche 56 als Stirnseite 54 auf dem Kontaktkörper 52, so dass die metallische Fläche 56 ein Mantelsegment des hülsenförmigen Kontaktkörpers 52 bildet. Die metallische Fläche 56 erstreckt sich z. B. über die ganze axiale Länge des Kontaktkörpers 52. Dessen weitere Oberfläche ist mit der elektrischen Isolation 58 abgedeckt.

Figur 15 zeigt eine alternative Ausgestaltung eines derartigen hülsenförmigen elektrischen Kontakts 50, bei dem die metallische Fläche 56 in einem abgeflachten Bereich 62 des Kontaktkörpers 52 angeordnet ist.

Zusätzlich können die in den Ausführungsbeispielen beschriebenen metallischen Flächen 56, 66 mit einer Oberflächenstruktur versehen sein, welche die Oberfläche der metallischen Flächen gegenüber ihren makroskopischen Abmessungen vergrößert.

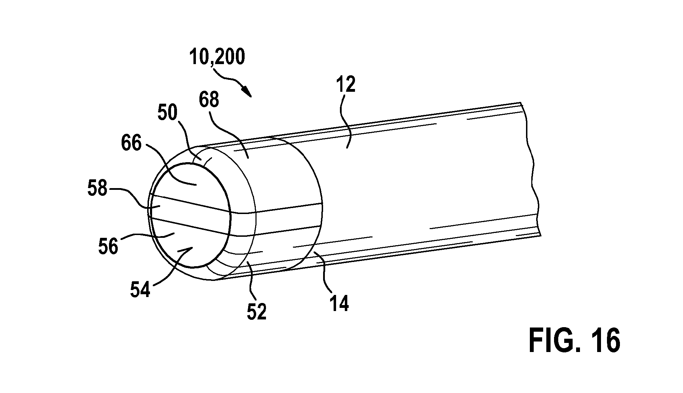

Figur 16 zeigt beispielhaft in isometrischer Darstellung ein Stimulationsaggregat 200 mit einem bipolaren stirnseitig angebrachten Elektrodenelement 10 mit zwei an der Stirnseite 54 des Kontaktkörpers 52 nebeneinander angeordneten metallischen Flächen 56, 66, der beispielsweise einen schnurlosen Herzschrittmacher darstellt. Beide metallischen Flächen 56, 66 bilden unterschiedliche elektrische Pole. Diese Elektrodenanordnung kann auch bei Herzelektroden angewendet werden. Der Kontaktkörper 52 des elektrischen Kontakts 50 ist an der Stirnfläche des länglichen Elementkörpers 12 angeordnet. An den Seitenwänden des Kontaktkörpers 52 ist eine elektrische Isolierung 68 vorgesehen. Die zwei nebeneinander angeordneten metallischen Flächen 56 sind ebenfalls durch eine elektrische Isolierung 58 voneinander elektrisch isoliert.

### Bezugszeichenliste

- 10: Elektrodenelement
- 12: Elementkörper
- 14: Spitze
- 16: Oberfläche
- 18: Öffnung

- 50: elektrischer Kontakt
- 52: Kontaktkörper
- 54: Stirnseite
- 56: metallische Fläche
- 58: elektrische Isolierung
- 59: elektrische Isolierung
- 69: Fläche
- 60: Bund
- 62: abgeflachter Bereich
- 66: metallische Fläche
- 68: elektrische Isolierung

- 100: Katheter mit Elektrodenelement
- 200: schnurloser Schrittmacher

## Patentansprüche

1. Elektrodenelement (10) zur elektromedizinischen Therapie in einem menschlichen oder tierischen Körper, umfassend einen Elementkörper (12), an dem wenigstens ein elektrischer Kontakt (50) an einer äußeren Oberfläche (16) des Elementkörpers (12) angeordnet ist, wobei der wenigstens eine elektrische Kontakt (50) aus der Oberfläche (16) des Elementkörpers (12) hervorsteht, **dadurch gekennzeichnet, dass** der wenigstens eine elektrische Kontakt (50) einen erhabenen Kontaktkörper (52) mit einer Stirnseite (54) aufweist, auf dem wenigstens eine metallische Fläche (56) ausgebildet ist, wobei die metallische Fläche (56) von einer elektrischen Isolierung (58) umgeben ist.

2. Elektrodenelement nach Anspruch 1, **dadurch gekennzeichnet, dass** mindestens Kanten um die metallische Fläche (56) mit der elektrischen Isolierung (58) wenigstens bereichsweise abgedeckt sind.

3. Elektrodenelement nach Anspruch 1 oder 2, **dadurch gekennzeichnet, dass** die elektrische Isolierung (58) wenigstens bereichsweise durch den Elektrodenkörper (12) gebildet ist.

4. Elektrodenelement nach einem der vorhergehenden Ansprüche, **dadurch gekennzeichnet, dass** der Kontaktkörper (52) als Ring ausgebildet ist und um den Elementkörper (12) gelegt ist.

5. Elektrodenelement nach Anspruch 4, **dadurch gekennzeichnet, dass** die elektrische Isolierung (58) in Umfangsrichtung mindestens die Hälfte des Rings bedeckt, so dass die metallische Fläche (56) ein Ringsegment bildet.

6. Elektrodenelement nach einem der vorhergehenden Ansprüche, **dadurch gekennzeichnet, dass** wenigstens zwei elektrische Kontakte (50) vorgesehen sind, die axial übereinander am Elementkörper (12) angeordnet sind, wobei vorzugsweise zwei elektrische Kontakte (50) unterschiedliche Polarität aufweisen.

7. Elektrodenelement nach einem der vorhergehenden Ansprüche, **dadurch gekennzeichnet, dass** wenigstens zwei elektrische Kontakte (50) vorgesehen sind, die axial und/oder radial voneinander beabstandet sind, wobei vorzugsweise zwei elektrische Kontakte (50) unterschiedliche Polarität aufweisen.

8. Elektrodenelement nach einem der vorhergehenden Ansprüche, **dadurch gekennzeichnet, dass** ein Array von mehreren elektrischen Kontakten (50) vorgesehen ist.

9. Elektrodenelement nach einem der vorhergehenden Ansprüche, **dadurch gekennzeichnet, dass** der wenigstens eine elektrische Kontakt (50) federnd gelagert ist.

10. Elektrodenelement nach einem der vorhergehenden Ansprüche, **dadurch gekennzeichnet, dass** der wenigstens eine elektrische Kontakt (50) mit einer Oberflächenstruktur ausgestattet ist, welche die effektive Kontaktfläche gegenüber makroskopischen geometrischen Abmessungen wenigstens verdoppelt.

11. Elektrodenelement nach einem der vorhergehenden Ansprüche, **dadurch gekennzeichnet, dass** der Kontaktkörper (52) wenigstens zwei getrennte metallische Flächen (56, 66) aufweist, wobei vorzugsweise die zwei getrennten metallischen Flächen (56, 66) für unterschiedlichen Polaritäten vorgesehen sind.

12. Elektrodenelement nach Anspruch 11, **dadurch gekennzeichnet, dass** die ersten der metallischen Flächen (66) im Wesentlichen eben ausgebildet ist, und die zweite der metallischen Flächen (56) über die erste metallische Fläche (66) hinausragt.

13. Elektrodenelement nach einem der vorhergehenden Ansprüche, **dadurch gekennzeichnet, dass** der Kontaktkörper (52) als Hülse ausgebildet ist, wobei die metallische Fläche (56) einen Teil der Mantelfläche der Hülse bildet.

14. Katheter mit einem Elektrodenelement (10) nach einem der vorhergehenden Ansprüche.

15. Stimulationsaggregat (200), insbesondere schurloser Schrittmacher oder Herzelektrode, mit einem Elektrodenelement (10) nach einem der Ansprüche 1 bis 13.
